# EUROPÄISCHE PATENTANMELDUNG

(11) **EP 0 945 507 A1**
(43) Veröffentlichungstag der Anmeldung: **29.09.1999**
(21) Anmeldenummer: 98105614.6
(22) Anmeldetag: 27.03.1998
(51) Int. Cl.: C12N 15/12, C07K 14/47, C12N 15/85, A61K 48/00

(54) **Tumorspezifische Expressionskontrollregion und deren Verwendung**

(71) Anmelder: Roche Diagnostics GmbH, 68305 Mannheim (DE)
(72) Erfinder: Die Erfindernennung liegt noch nicht vor

(57) **Zusammenfassung**

Ein Expressionsvektor, enthaltend ein Gen, welches für ein in Tumorzellen therapeutisch wirksames Transkriptions- oder Translationsprodukt codiert, wobei dieses Gen unter der Kontrolle einer Expressionskontrollregion mit der Sequenz SEQ ID NO:1 oder einem Fragment davon, welches mindestens die Basen bp -224 bis -197 aus SEQ ID NO:1 umfaßt, ist tumorzellspezifisch und für die in vivo und in vitro Ablation oder Regression von Tumorzellen geeignet.

## Beschreibung

Die Erfindung betrifft eine tumorspezifische Expressionskontrollregion, diese enthaltende Vektoren und deren Verwendung, insbesondere zur in vivo Expression.

Die spezifische Expression von tumoriziden Fremdgenen in Tumoren ist ein vielversprechender Ansatz in der therapeutischen Behandlung von Tumorerkrankungen.

Aus Bosserhoff, A.K., et al., J. Biol. Chem. 271 (1996) 490-495 ist die Struktur und die Promotoranalyse des Gens, welche das humane Melanoma-inhibierende Protein MIA (auch als CD-RAP bezeichnet) codiert, bekannt. MIA wird in Melanomzellinien exprimiert und zeigt Wachstums-inhibierende Effekte auf Melanomazellen in vitro (Bogdahn et al., Cancer Res. 49 (1989) 5358-5363; Internationale Anmeldung Nr. WO 95/03328). Aus Bosserhoff und der WO 95/03328 ist weiter bekannt, daß eine Region von etwa 500 Basenpaaren der 5 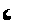 -untranslatierten Region des MIA-Gens die Expression von MIA in malignen Melanomzellen bewirkt.

Aus Kondo, S., et al., 44^{th} Annual Meeting, Orthopaedic Research Society, March 16-19, 1998, New Orleans, Louisiana, S. 178-30 ist bekannt, daß IGF-I die CD-RAP-Gen-Expression über eine AP-2-Bindungsstelle (bp -475 bis -458) reguliert.

Aus Xie, W.F., 44^{th} Annual Meeting, Orthopaedic Research Society, March 16-19, 1998, New Orleans, Louisiana, S. 207-35 ist bekannt, daß ein 2,2 kb CD-RAP-Promotorfragment die Expression von lacZ in transgenen Mäusen bewirkt. Eine Blaufärbung zeigt, daß alle Regulationselemente von CD-RAP vorhanden sind, um die natürliche CD-RAP-Expression zu ermitteln.

Aufgabe der Erfindung ist es, den MIA-Promotor so zu verbessern, daß er in der Lage ist, in vivo hochspezifisch in Tumorzellen Gene zu exprimieren.

Die Aufgabe wird gelöst durch einen tumorzellspezifischen Expressionsvektor, enthaltend ein Gen, welches für ein in Tumorzellen therapeutisch wirksames Transkriptions- oder Translationsprodukt codiert, wobei dieses Gen unter der Kontrolle einer Expressionskontrollregion der Sequenz SEQ ID NO:1 oder einem Fragment davon, welches mindestens bp -224 bis -197 aus SEQ m NO:1 umfaßt, steht.

Die Angaben zur Zählweise der Basen (z.B. bp -224) entsprechen der dem Fachmann geläufigen Zählweise von Expressionskontrollsequenzen (Upstream-Zählweise). Dabei gilt folgende Zuordnung:

| SEQ ID NO:1 | Upstream-Zählweise |
|---|---|
| 1 | -380 |
| 157 | -224 |
| 184 | -197 |
| 380 | -1 |

Unter 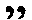 bp -224 aus SEQ ID NO:1" wird demnach gemäß der Erfindung im Einzel- oder Doppelstrang die Base oder das Basenpaar (bp) 157 aus SEQ ID NO: 1 verstanden.

Es hat sich überraschenderweise gezeigt, daß ein solcher Vektor eine tumorspezifische Expression von therapeutisch wirksamen Genen in Tumorzellen ermöglicht.

Unter einer Expressionskontrollregion ist eine Nukleinsäureregion zu verstehen, welche die Expression von DNA und damit die Transkription zu mRNA bewirkt. Solche Expressionskontrollregionen enthalten üblicherweise Enhancerregionen und Promotorregionen, an welche Transkriptionsfaktoren oder Repressoren binden können. Expressionskontrollregionen können über Bindung von aktivierenden oder reprimierenden Faktoren reguliert werden.

Eine solche Expressionskontrollregion enthält mindestens das Nukleinsäurefragment (Oligonukleotid) bp -224 bis -197 aus SEQ ID NO:1 oder aktive regulatorische Teile davon. Ein solches Oligonukleotid enthält zwei hochkonservierte Bindungsstellen, Region X (bp - 197 bis -207) und Region Y (bp -224 bis -214). Diese regulatorischen Nukleinsäurefragmente (regulatorische Region) sind auch in Kombination mit anderen Promotoren wie beispielsweise dem TK Promotor, dem Minimal Early SV40 oder dem CMV Immediate Early Promotor in Expressionsvektoren ebenfalls als erfindungsgemäße Expressionskontrollegion geeignet.

Unter einer regulatorischen Region ist eine Region zu verstehen, welche die Expression in negativer oder positiver Weise beeinflußt. Falls in der erfindungsgemäßen Expressionskontrollsequenz eine negativ regulierende Region enthalten ist, so wird die tumorspezifische Expression dadurch erreicht, daß in Tumorzellen eine solche Inhibition aufgehoben ist und vice versa. Analog kann die tumorspezifische Expression durch Elemente inhibiert werden, welche an eine positiv regulierende Region binden (Antisense).

Wesentlich für eine tumorspezifische Expression ist es, daß der Abstand der regulatorischen Nukleinsäurefragmente Region X und Region Y nicht allzu groß ist. Vorzugsweise beträgt der Abstand zwischen 0 und 20 bps.

Als Expressionskontrollregion ist sowohl die in SEQ ID NO: 1 beschriebene humane MIA-Region als auch entsprechende (homologe) MIA-Regionen von Säugetieren wie beispielsweise Maus, Ratte, geeignet Die humane MIA-Sequenz ist in der EMBL Datenbank unter Nummer X84707, die murine Sequenz unter Nummer 485612 beschrieben.

Unter einem therapeutisch wirksamen Translationsprodukt ist ein Polypeptid (Protein) zu verstehen, welches in Tumorzellen eine Regression oder eine Ablation unmittelbar hervorruft oder auch eine solche über eine Stimulation des Immunsystems bewirkt. Geeignete Gene codieren beispielsweise für Tumorsuppressorproteine, für Proteine, welche Apoptose induzieren (z.B. p53), Pro-Drug-Aktivatoren (Suizidgene, wie TK oder Cytosindesaminase (CDA)), Immunstimulatoren (z.B. Cytokine), Costimulatoren (z.B B7-1 oder B7-2) oder toxische Proteine, wie beispielsweise Choleratoxin.

Unter einem therapeutisch wirksamen Transkriptionsprodukt ist vorzugsweise eine Antisense-Sequenz (z.B. Ribozyme oder Antisense-RNA) zu verstehen, welche gegen ein Onkogen, ein die Apoptose hemmendes Gen oder ein anderes Tumorgen wie beispielsweise MIA (WO 95/03328) gerichtet ist. Ein solches Transkriptionsprodukt ist dadurch therapeutisch wirksam, daß es in den Tumorzellen eine Regression oder eine Ablation verursacht. Dies kann beispielsweise durch Inhibition der Expression eines Tumorgens oder eines Onkogens erfolgen.

Solche therapeutisch wirksamen Transkriptions- oder Translationsprodukte unterscheiden sich damit von sogenannten Indikatorgenen, wie dem CAT- oder Luciferase-Gen, welche aus Prokaryonten oder Insekten stammen und in Säugerzellen keine therapeutische Wirksamkeit haben und lediglich zur Expressionskontrolle und zum Expressionsnachweis dienen.

Der erfindungsgemäße Expressionsvektor ist überraschenderweise Tumor- bzw. tumorzellspezifisch, insbesondere spezifisch für Metastasenzellen, da der MIA-Promotor insbesondere in solchen Tumorzellen aktiv ist, die aus dem Tumorverband abgelöst und damit mobilisiert sind.

Das therapeutisch wirksame Transkriptionsprodukt, welches vorzugsweise eine Antisense-Nukleinsäure ist, bindet in vitro unter stringenten Bedingungen an eine Nukleinsäure der Sequenz SEQ ID NO:1. Solche stringenten Standardbedingungen und Verfahren zur Hybridisierung sind dem Fachmann bekannt und beispielsweise von Sambrook, J., et al. in "Expression of cloned genes in E. coli" in Molecular Cloning: A laboratory manual (1989), Cold Spring Harbor Laboratory Press, New York, USA und Hames, B.D., und Higgins, S.J., in Nucleic Acid Hybridisation - A Practical Approach, Hrsg. Hames und Higgins (1985), IRL Press beschrieben. Die dort beschriebenen Standardprotokolle werden üblicherweise hierfür verwendet. Insbesondere wird hingewiesen auf Sambrook, Section IX.

Bevorzugte stringente Bedingungen sind gegeben bei einer Hybridisierung in Gegenwart von 1 mol/l NaCl, 1% SDS und 10% Dextransulfat bei anschließendem zweifachen Waschen des Filters bei Raumtemperatur für 5 Minuten in 2 x SSC und einem Waschschritt für 30 Minuten. Dieser weitere Waschschritt kann bei 0.5 x SSC, 0,1% SDS, vorzugsweise bei 0,2 x SSC und 0,1% SDS und besonders bevorzugt bei 0,1 x SSC, 0,1% SDS bei 65°C durchgeführt werden.

Ein weiterer Gegenstand der Erfindung ist ein Verfahren zur Herstellung eines erfindungsgemäßen tumorspezifischen Expressionsvektors, wobei in einem geeigneten Vektor ein Gen so inseriert wird, daß es unter der Kontrolle der erfindungsgemäß beschriebenen Expressionskontrollregion exprimiert wird. Bei solchen Vektoren handelt es sich zweckmäßig um Vektoren, welche für die Gentherapie geeignet sind. Solche Vektoren können entweder nackte oder formulierte Plasmid-DNA (z.B. mit Tranferreagenzien, wie Liposomen, formuliert) oder virale Nukleinsäuren darstellen.

Die erfindungsgemäß hergestellten tumorzellspezifischen Expressionsvektoren können vorteilhaft zur Regression oder Ablation von Tumoren und Tumorzellen, vorzugsweise von Melanom-, Colonkarzinom- und/oder Mammakarzinomzellen und davon abgeleiteten metastasierenden Zellen, ex vivo oder in vivo zur Gentherapie verwendet werden. Die Regression oder Ablation von Tumorzellen kann unmittelbar (beispielsweise durch Expression von p53) oder mittelbar (durch Expression von immunstimulierenden Agenzien, wie beispielsweise Cytokinen, wie IL-2, GM-CSF oder IL-12) erfolgen.

Ein weiterer Gegenstand der Erfindung ist ein Verfahren zur Herstellung eines pharmazeutischen Mittels zur Regression oder Ablation von Primärtumoren, Residualtumoren, Metastasen und Minimal Residual Disease in vivo oder ex vivo von Tumor- und Leukämiezellen, welches als wesentlichen Bestandteil einen erfindungsgemäßen Expressionsvektor enthält.

Ein weiterer Gegenstand der Erfindung ist ein Nukleinsäurefragment mit einer Länge von 10 bis 28 Basen aus dem Bereich bp -224 bis -197 aus SEQ ID NO:1 oder der dazu komplementären Nukleinsäure unter stringenten Bedingungen hybridisiert. Ein solches Nukleinsäurefragment ist als tumorzellspezifisches regulatorisches Element in einer Expressionskontrollsequenz geeignet. Ebenso geeignet ist ein solches Nukleinsäurefragment zum Nachweis und zur Identifikation von an dieses Nukleinsäurefragment bindenden Substanzen. Solche Nachweisverfahren sind beispielsweise beschrieben in US-Patent Nr. 5,578,444, US-Patent Nr. 5,716,760 und dem kanadischen Patent Nr. 2,112,130. Dabei wird das Nukleinsäurefragment an die DNA kovalent gebunden, und ein Bindungspartner des Nukleinsäurefragments beeinflußt die DNA-Proteinbindung.

Ein weiterer Gegenstand der Erfindung basiert auf der Bindung von Elementen, die komplementär an das Nukleinsäurefragment (bp - 197 bis -224) binden können (vorzugsweise unter physiologischen Bedingungen, z.B. in Tumorzellen), um die Expression des MIA-Promotors zu inhibieren. Solche Elemente sind beispielsweise Antisense-RNA, Ribozyme, PNA oder Chimeraplasten und können sinngemäß wie oben beschrieben modifiziert und/oder direkt (ohne daß als Vehikel ein Expressionsvektor nötig ist) eingesetzt werden.

Ein weiterer Gegenstand der Erfindung ist demnach ein erstes Nukleinsäurefragment, welches an ein zweites Nukleinsäurefragment der Sequenz SEQ ID NO:1 oder ein Fragment davon, welches mindestens eine Base bp -224 bis -197 aus SEQ ID NO:1 umfaßt, unter physiologischen Bedingungen spezifisch bindet und mit dem doppelsträngigen zweiten Nukleinsäurefragment im Bereich der Basen bp -224 bis -197 aus SEQ ID NO:1 eine Tripelhelix bildet oder in das zweite Nukleinsäurefragment im Bereich der Basen bp -224 bis -197 aus SEQ ID NO:1 eine oder mehrere Punktmutationen einführt. Ein solches erstes Nukleinsäurefragment kann zur Inhibition der MIA-Expression in Tumorzellen verwendet werden.

Das Nukleinsäurefragment (vorzugsweise Faktor X) stellt ein konserviertes Expressionskontrollelement dar. Die Mutation von wenigen Basen (vorzugsweise 1 bis 3 Basen) inhibiert oder verringert die Expressionsaktivierung des downstream gelegenen Promotorelements. Es ist deshalb bevorzugt, zur therapeutischen Anwendung Antisense-RNA, Ribozyme, PNA oder Chimeraplasten in Tumorzellen einzuführen, welche eine derartige Inhibition oder Mutation im MIA-Promotor bewirken und dadurch die MIA-Expression in solchen Zellen inhibieren und dadurch wiederum das Metastasierungspotential dieser Tumorzellen (vorzugsweise Melanoma, Mammakarzinom, Colonkarzinom) verringern oder aufheben.

Unter einem Chimeraplasten ist gemäß der Erfindung ein chimäres DNA-RNA-Hybridmolekül (zweckmäßig 60 bis 90 bp) zu verstehen, welches in der Lage ist, sequenzspezifisch an DNA zu binden und die oben beschriebenen Punktmutationen durchzuführen. Solche Chimeraplasten und deren Herstellung sind im US-Patent Nr. 5,565,350 beschrieben. Die in vivo-Anwendung ist von Kren, B.T., et al., Nature Medicine 4(1998)285-290 beschrieben.

Ein erfindungsgemäßer Expressionsvektor kann ebenfalls vorteilhaft für ein ex vivo Purging von Leukämiezellen oder Tumorzellen bei der autologen Knochenmarkstransplantation verwendet werden. Da der Expressionsvektor spezifisch in Tumorzellen aktiv ist, können damit in autologen Transplantationspräparaten sonst nicht erkennbare Tumorzellen markiert werden. Dies geschieht zweckmäßig dadurch, daß ein geeigneter Tumorzellenmarker, wie beispielsweise in WO 95/06723 beschrieben, verwendet wird. Besonders vorteilhaft wird das LNGFR-Gen als Markierungsgen für Tumorzellen aus autologen Knochenmarks-Transplantationspräparaten verwendet. Zusätzlich wird ein erfindungsgemäßer Expressionsvektor, der in diesem Fall nicht für ein therapeutisch wirksames Produkt codiert, sondern für ein selektierbares Indikatorgen, verwendet. Das bevorzugte LNGFR-Gen exprimiert den LNGFR-Rezeptor, der die Zelloberfläche markiert und zuverlässig über Antikörper erkannt wird. Damit kann eine Entfernung der unerwünschten Tumorzellen aus den autologen Knochenmarkspräparaten erfolgen. Analog können auch Suizidgene, Toxingene oder Apoptose-induzierende Gene eingebracht und zum Abtöten der Tumor- oder Leukämiezellen zum Purging verwendet werden.

Die folgenden Beispiele, Publikationen, das Sequenzprotokoll und die Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Beschreibung der Figuren

- **Fig. 1**: Restriktionskarte von Plasmid pCMVh12-bgh-cat.
- **Fig. 2**: Restriktionskarte von Plasmid pLT1.
- **Fig. 3**: Restriktionskarte von Plasmid pCMVhi12ireshb7-1.

### Beispiel 1

### Konstruktion der Expressionsvektoren

### 1.1. Cat-Reportergen als Kontrolle:

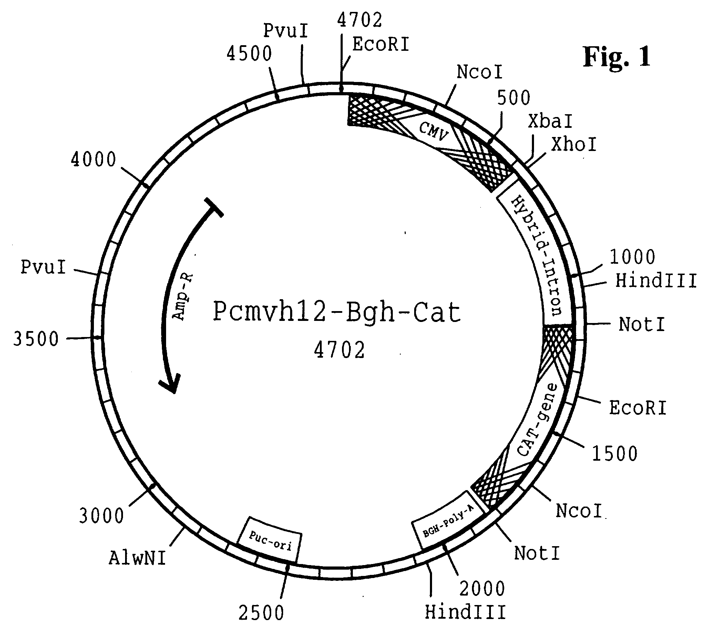

Basierend auf dem Reportergenplasmid pCMVh12-bgh-cat (Fig. 1) wird das Konstrukt pMIA380h12-bgh-cat wie folgt hergestellt: Aus pCMVh12-bgh-cat wird der CMV-Promoter durch einen PstI/XhoI-Doppelverdau entfernt. Das verkürzte MIA-Promoterfragment (0 bis - 380 bp) wird aus pBL-MIA1386 (welches die gesamte in Bosserhoff, A.K., et al., J. Biol. Chem. 271(1996) 490-495 beschriebene MIA-Promotorregion enthält) mittels PCR mit entsprechenden Primern, die überhängende Enden mit den PstI/XhoI-Sites tragen, amplifiziert und in die PstI/XhoI-Schnittstelle kloniert.

### 1.2. Prodrug-aktivierbares Suizidgen HSV-TK:

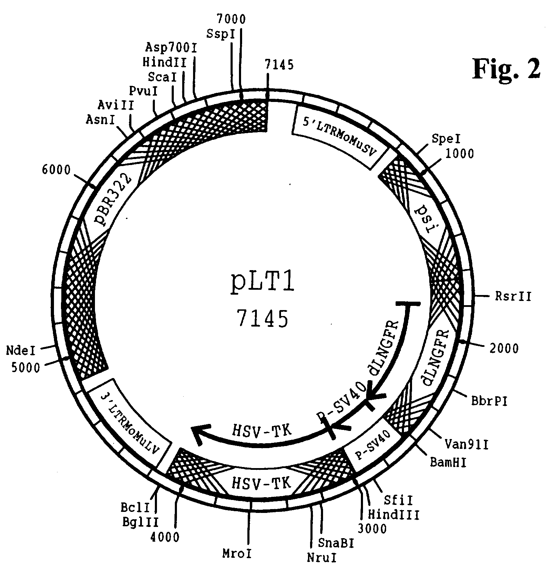

Basierend auf dem Konstrukt pMIA380h12-bgh-cat wird das Konstrukt pMIA380h12-bgh-HSV-TK wie folgt hergestellt: Aus pMIA380h12-bgh-cat wird das Cat-Gen durch einen NotI-Verdau entfernt. Das HSV-TK Gen wird aus pLT1 (Fig. 2) mittels PCR und Primern gegen das 5' und das 3'-Ende des Gens, die überhängende Enden mit der NotI-Site tragen, amplifiziert und in die NotI-Schnittstelle kloniert.

### 1.3. Immunstimulatorisches IL-2 Gen:

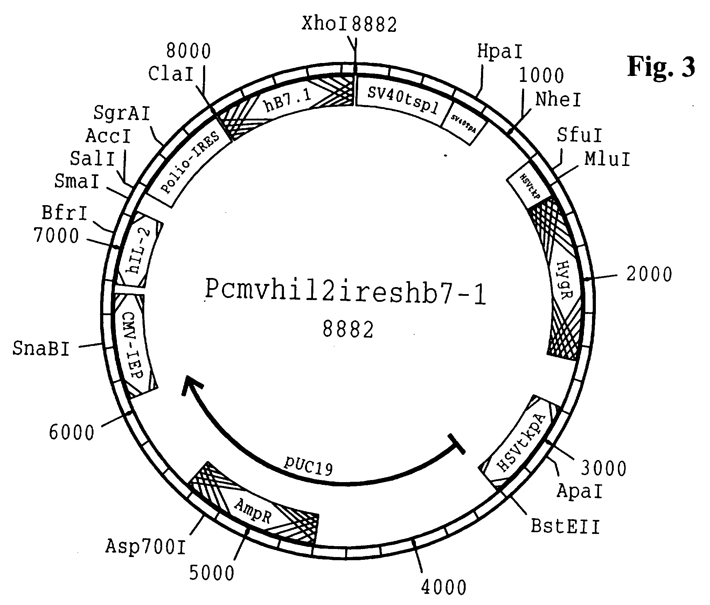

Basierend auf dem Konstrukt pMIA380h12-bgh-cat wird das Konstrukt pMIA380h12-bgh-hIL-2 wie folgt hergestellt: Aus pMIA380h12-bgh-cat wird das Cat-Gen durch einen NotI-Verdau entfernt. Aus pCMVhIL2IREShB7-1 (Fig. 3) wird das humane IL-2 Gen mittels PCR und Primern gegen das 5' und das 3'-Ende der cDNA, die überhängende Enden mit den NotI-Sites tragen, amplifiziert und in die NotI-Schnittstelle kloniert.

### 1.4. Immunstimulatorisches GM-CSF-Gen:

Basierend auf dem Konstrukt pMIA380h12-bgh-cat wird das Konstrukt pMIA380h12-bgh-GM-CSF wie folgt hergestellt: Aus pMIA380h12-bgh-cat wird das Cat-Gen durch einen NotI-Verdau entfernt. GM-CSF ist in EP-B 0 202 300 und EP-B 0 188 479 beschrieben (siehe auch (Dranoff, G., et al., Proc. Natl. Acad. Sci. USA 90 (1993) 3539-3543). Das GM-CSF-Gen wird mittels PCR und Primern gegen das 5' und das 3'-Ende der cDNA, die überhängende Enden mit den NotI-Sites tragen, amplifiziert und in die NotI-Schnittstelle kloniert.

### Beispiel 2

### Transfektion der oben genannten Vektoren in murine B16-Melanomazellinie

Die murine B16 Melanomazellinie (ATCC# CRL 6322) wird in DMEM + 10% FKS und L-Glutamin kultiviert. Als Transfektionsreagens wird DOSPER (Boehringer Mannheim GmbH, Mannheim, DE) nach Herstellerangaben verwendet; Transfektionsmedium serumfrei.

### pCMVh12-bgh-cat bzw. pMIA380h12-bgh-cat:

Die B 16 Zellen werden transfiziert; nach 2 - 3 Tagen wird die Cat-Aktivität gemessen. Der Test auf Cat-Aktivität wird mit einem Cat-ELISA (Boehringer Mannheim GmbH, Mannheim, DE) nach Herstellerangaben durchgeführt und die für das verkürzte MIA-Promotorfragment (Beispiel 1.1) erhaltenen Ergebnisse mit denen des CMV-Promotors verglichen.

### pCMVh12-bgh-IL-2 bzw. pMIA380h12-bgh-IL-2:

Die B 16 Zellen werden transfiziert; nach 2 - 3 Tagen wird die IL-2-Aktivität gemessen. Der Test auf IL-2 wird mit einem IL-2 ELISA (Boehringer Mannheim GmbH, Mannheim, DE) nach Herstellerangaben durchgeführt und die für das verkürzte MIA-Promotorfragment (Beispiel 1.1) erhaltenen Ergebnisse mit denen des CMV-Promotors verglichen.

### pCMVh12-bgh-HSV-TK bzw. pMIA380h12-bgh-HSV-TK:

Die Transfektion in die B 16-Zellen wird mit Asp700I-linearisiertem Plasmid durchgeführt. Das Plasmid pCDNA3 (Invitrogen), welches eine NeoR-Expressionskassette enthält, wird im 10-fachen molaren Unterschuß kotransfiziert. Selektioniert wird mit 50 µg/ml G418 für 2 - 3 Wochen. Anschließend werden durch Limited Dilution und PCR-Screening der erhaltenen Einzelklone HSV-TK-Gen positive B16-Melanomazellklone isoliert.

Der in vitro-Test auf HSV-TK-Aktivität wird mit Ganciclovir (Cymeven®, Syntex / 10 µg/ml (Beck, C., et al., Human Gene Therapy 6 (1995) 1525-1530) durchgeführt; HSV-TK exprimierende, positive Zellen sterben ab. Die für das verkürzte MIA-Promotorfragment (Beispiel 1.1) erhaltenen Ergebnisse werden mit denen des CMV-Promotors verglichen.

Von **pMIA380h12-bgh-GM-CSF** wird analog zu dem HSV TK-Gen eine stabile B16-Melanomazellinie angelegt; die positiven Klone werden durch PCR und einen GM-CSF-ELISA (Endogen) charakterisiert.

### Beispiel 3

### Injektion der stabilen pMIA380h12-bgh-HSV-TK und pMIA380h12-bgh-GM-CSF transfizierten B16-Melanomalinie in syngene C57B16 Mäuse

### HSV-TK:

1 x 10⁶ B16/HSV-TK-Zellen werden in PBS gewaschen und subcutan in einem Volumen von 200 µl in die Abdominalwand der C57B16 Mäuse (Fidler, I.J., Cancer Research 35 (1975) 218-224) injiziert. Nach 4 - 6 Tagen ist der stabil transfizierte Tumor angewachsen.

Variante: 1 x 10⁵ B16/HSV-TK-Zellen werden in PBS gewaschen und intravenös in die C57B16-Mäuse injiziert. Nach 10 - 14 Tagen sind die Lungenmetastasen angewachsen.

Durch GCV-Gaben (2 x täglich; über 5 Tage; 150 mg/kg GCV in 200 µl 0,9 %-iger NaCl-Lösung (Beck, C., et al., Human Gene Therapy 6 (1995) 1525-1530) bzw. als Kontrolle nur 0,9%-ige NaCl-Lösung) werden die HSV-TK exprimierenden, nicht aber die nichtexprimierenden B16-Melanome abgetötet. Die Mäuse werden getötet, seziert und histologisch untersucht; die GCV-behandelten Tiere mit nichtbehandelten Tieren verglichen.

### GM-CSF(3):

Die syngenen C57B16-Mäuse werden durch subcutane Injektion von 5 x 10⁵ lebenden, pMIA380h12-bgh-GM-CSF transfizierten B16-Zellen in den Abdomen vakziniert. Nach 7 - 14 Tagen werden die Tiere mit 5 x 10⁵ lebenden, nichttransduzierten B16-Melanomazellen durch subcutane Injektion in den Rücken gechallenged. Die Mäuse werden getötet, seziert und untersucht, wenn 2 - 3 cm große Tumoren auftreten bzw. nach längstens 100 Tagen. Die Mäuse, die eine Injektion von stabil mit dem GM-CSF-Gen unter MIA-Promotorkontrolle transfizierten B16-Zellen erhalten haben, werden mit Mäusen, die untransfizierte B16-Zellen erhalten haben, verglichen.

### Beispiel 4

### Injektion der Plasmide pMIA380h12-bgh-HSV-TK und pMIA380h12-bgh-GM-CSF als Formulierung mit DOTAP (Boehringer Mannheim GmbH, DE) in etablierte B16-Melanomatumoren bzw. in normales Muskelgewebe der syngenen C57B16-Mäuse

### HSV-TK:

1 x 10⁶ B16-Zellen werden in PBS gewaschen und subcutan in einem Volumen von 200 µl in die Abdominalwand der syngenen C57B16 Mäuse injiziert. Nach 4 - 6 Tagen ist der stabil transfizierte Tumor angewachsen. pMIA380h12-bgh-HSV-TK wird mit DOTAP formuliert in die präformierten B16-Melanomtumoren injiziert bzw. in gesundes Muskelgewebe. Nach 2 - 3 Tagen wird Ganciclovir gegeben (2 x täglich; über 5 Tage; 150 mg/kg GCV in 200 µl 0,9%-ige NaCl-Lösung bzw. als Kontrolle nur 0,9 %-ige NaCl-Lösung); die durch das HSV-TK-Gen transfizierten und daher HSV-TK exprimierenden Tumorzellen werden durch die Prodrugaktivierung abgetötet, nicht aber die nichtexprimierenden Zellen (normale Körperzellen bzw. normale Körperzellen, die durch pMIA380h12-bgh-HSV-TK transduziert wurden, in denen der MIA-Promoter jedoch nicht aktiviert wird, d.h. ebenfalls kein HSV-TK exprimiert wird). Die Mäuse werden getötet, seziert und untersucht; die GCV-behandelten Tiere mit nichtbehandelten Tieren histologisch verglichen. Ebenfalls werden die behandelten Gewebe über PCR und RT-PCR auf Vorhandensein bzw. Expression des transgenen HSV-TK Gens verglichen.

### GM-CSF:

1 x 10⁶ B16-Zellen werden in PBS gewaschen und subcutan in einem Volumen von 200 µl in die Abdominalwand der syngenen C57B16 Mäuse injiziert. Nach 4 - 6 Tagen ist der stabil transfizierte Tumor angewachsen. pMIA380h12-bgh-GM-CSF wird mit DOTAP formuliert in die präformierten B16-Melanomatumoren injiziert bzw. in Muskelgewebe. Nach 7 - 14 Tagen werden die Mäuse getötet, seziert und auf immunstimulatorische Wirkung des GM-CSF-Gens histologisch (Tumorgröße, Makrophageninfiltration) bzw. auf Vorhandensein der Plasmid-DNA über PCR bzw. auf GM-CSF-Expression über RT-PCR untersucht. Die Mäuse, welche pMIA380h12-bgh-GM-CSF / DOTAP in die präformierten B16-Melanomatumoren erhalten haben, werden mit Mäusen, welche nur leeres Vektorplasmid bzw. eine Injektion in Muskelgewebe erhalten haben, verglichen.

### Beispiel 5

### Einfluß von Mutationen in der Region X auf die MIA-Expression

Durch Site-Directed-Mutagenesis (Site Directed Mutagenesis-Kit, Clontech) werden in Region X der Expressionskontrollsequenz gemäß Tabelle 1 Mutationen in ein Reporterplasmid, welches das Luciferasegen unter der Kontrolle des MIA-Promotorfragments gemäß SEQ ID NO:1 enthält, eingeführt und die Expression in malignen Melanomzellen (MM, B16) und Nicht-Melanomzellen (nonMM, HeLa) geprüft. Tabelle 1 zeigt das Ergebnis.

### Zur Herstellung des Reporterplasmids wird das MIA-Promotrfragment sowie das Luciferase-Indikatorgen über HindIII/BgIII in den Vektor pGL3 basic (Promega GmbH, Mannheim, DE) inseriert.

**Tabelle 1**

| **Einfluß von Mutationen in der Region X auf die MIA-Expression** | | | | | | |
|---|---|---|---|---|---|---|
| Codons aus Region X | | | | | MM | non-MM |
| | TAG | GCA | TTT | TCT | +++ | - |
| Mut 1 | --X | -XX | XXX | --- | - | - |
| Mut 3 | --- | X-X | --- | --- | - | - |
| Mut 4 | --X | X-X | --- | --- | - | - |
| Mut 5 | --X | --- | --- | --- | ++ | - |
| Mut 6 | --- | --X | --- | --- | + | - |

### Referenzliste

Beck, C., et al., Human Gene Therapy 6 (1995) 1525-1530
Bogdahn et al., Cancer Res. 49 (1989) 5358-5363
Bosserhoff, A.K., et al., J. Biol. Chem. 271 (1996) 490-495
CA-Patent Nr. 2,112,130
Dranoff, G., et al., Proc. Natl. Acad. Sci. USA 90 (1993) 3539-3543
EP-B 0 188 479
EP-B 0 202 300
Fidler, I.J., Cancer Research 35 (1975) 218-224
Hames, B.D., und Higgins, S.J., in Nucleic Acid Hybridisation - A Practical Approach, Hrsg. Hames und Higgins (1985), IRL Press
Kondo, S., et al., 44^{th} Annual Meeting, Orthopaedic Research Society, March 16-19, 1998, New Orleans, Louisiana, S. 178-30
Kren, B.T., et al., Nature Medicine 4 (1998) 285-290
Sambrook, J., et al. in "Expression of cloned genes in E. coli" in Molecular Cloning: A laboratory manual (1989), Cold Spring Harbor Laboratory Press, New York, USA
US-Patent Nr. 5,565,350
US-Patent Nr. 5,578,444
US-Patent Nr. 5,716,760
WO 95/03328
WO 95/06723
Xie, W.F., 44^{th} Annual Meeting, Orthopaedic Research Society, March 16-19, 1998, New Orleans, Louisiana, S. 207-35

## Patentansprüche

1. Tumorzellspezifischer Expressionsvektor, enthaltend ein Gen, welches für ein in Tumorzellen therapeutisch wirksames Transkriptions- oder Translationsprodukt codiert, wobei dieses Gen unter der Kontrolle einer Expressionskontrollregion mit der Sequenz SEQ ID NO:1 oder einem Fragment davon, welches mindestens die Basen bp -224 bis -197 aus SEQ ID NO:1 umfaßt, steht.

2. Expressionsvektor nach Anspruch 1, dadurch gekennzeichnet, daß das therapeutisch wirksame Transkriptions- oder Translationsprodukt eine Tumorregression, Tumorablation oder Immunstimulation in Tumorzellen bewirkt.

3. Expressionsvektor nach Anspruch 1, dadurch gekennzeichnet, daß das therapeutisch wirksame Transkriptionsprodukt eine Antisense-Nukleinsäure oder ein Ribozym ist, welche in vitro unter stringenten Bedingungen an eine Nukleinsäure der Sequenz SEQ ID NO:1 binden.

4. Expressionsvektor nach Anspruch 1 oder 2, dadurch gekennzeichnet, daß das therapeutisch wirksame Translationsprodukt ein Apoptose-induzierendes, Tumor-supprimierendes, immunstimulierendes, costimulatorisches oder toxisches Polypeptid ist.

5. Verfahren zur Herstellung eines tumorspezifischen Expressionsvektors nach den Ansprüchen 1 bis 4, dadurch gekennzeichnet, daß in einem Vektor das therapeutisch wirksame Gen so inseriert wird, daß es unter der Kontrolle der Expressionskontrollregion exprimiert wird.

6. Verwendung eines Expressionsvektors nach den Ansprüchen 1 bis 4 zur Regression oder Ablation von Primärtumoren, Residualtumoren, Metastasen und Minimal Residual Disease in vivo oder ex vivo von Tumor- und Leukämiezellen.

7. Verfahren zur Herstellung eines pharmazeutischen Mittels zur Regression oder Ablation von Primärtumoren, Residualtumoren, Metastasen und Minimal Residual Disease in vivo oder ex vivo von Tumor- und Leukämiezellen, dadurch gekennzeichnet, daß als wesentlicher Bestandteil des Mittels ein Expressionsvektor nach den Ansprüchen 1 bis 4 verwendet wird.

8. Nukleinsäurefragment mit einer Länge von 10 bis 30 Basen, dadurch gekennzeichnet, daß das Fragment mit der Basenfolge -224 bis -197 aus SEQ ID NO:1 oder einer dazu komplementären Nukleinsäure unter stringenten Bedingungen hybridisiert.

9. Verwendung eines Nukleinsäurefragments der Sequenz SEQ ID NO:1 oder einem Fragment davon, welches mindestens die Basen bp -224 bis - 197 aus SEQ ID NO:1 umfaßt, in einem Verfahren zum Nachweis einer DNA-Proteinbindung, dadurch gekennzeichnet, daß das Nukleinsäurefragment an die DNA kovalent gebunden ist und ein Bindungspartner des Nukleinsäurefragments die DNA-Proteinbindung beeinflußt.

10. Erstes Nukleinsäurefragment, welches an ein zweites Nukleinsäurefragment der Sequenz SEQ ID NO:1 oder einem Fragment davon, welches mindestens die Basen bp -224 bis - 197 aus SEQ in NO: 1 umfaßt, unter physiologischen Bedingungen spezifisch bindet und mit dem doppelsträngigen zweiten Nukleinsäurefragment im Bereich der Basen bp -224 bis -197 aus SEQ in NO: 1 eine Tripelhelix bildet oder in das zweite Nukleinsäurefragment im Bereich der Basen bp -224 bis -197 aus SEQ ID NO:1 eine oder mehrere Punktmutationen einführt.

11. Verwendung eines ersten Nukleinsäurefragments gemäß Anspruch 10 zur Inhibition der MIA-Expression in Tumorzellen.


SEQUENZPROTOKOLL

(1) ALLGEMEINE ANGABEN:

(i) ANMELDER:
(A) NAME: BOEHRINGER MANNHEIM GMBH
(B) STRASSE: Sandhofer Str. 116
(C) ORT: Mannheim
(E) LAND: Germany
(F) POSTLEITZAHL: D-68305
(G) TELEFON: 08856/60-3446
(H) TELEFAX: 08856/60-3451

(ii) BEZEICHNUNG DER ERFINDUNG: Tumorspezifische Expressions-
kontrollregion und deren Verwendung

(iii) ANZAHL DER SEQUENZEN: 1

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30B (EPA)

(2) ANGABEN ZU SEQ ID NO: 1:

(i) SEQUENZKENNZEICHEN:
(A) LÄNGE: 383 Basenpaare
(B) ART: Nucleotid
(C) STRANGFORM: Doppelstrang
(D) TOPOLOGIE: linear

(ii) ART DES MOLEKÜLS: other nucleic acid
(A) BESCHREIBUNG: /desc = "Expressionskontrollregion"
Base 1 = -380
Base 380 = -1

(xi) SEQUENZBESCHREIBUNG: SEQ ID NO: 1:

CTTTACAGGC TCCTCCGCTT CTGTGGCCAG AGGGGACAGC GGAGGACCCC AGGTACCTAA 60

GCCAACTCAA GAGAAGATGG AATTGAATAT TTCAACCACC TTATCTAGGC CTCTGTGATT 120

GTTGAGGAGG GGGCTGTCAC TGGGAAAGTT GTGAGCTGCT TTGGACCTTA TCTGGGAATT 180

TCCTTGGGCC TTACAGCTTT ACCCTATCCT TGAAATGGTT CTGGTTTCAT AGCAACTTCT 240

AGGTGGTGTG GGCGAAGTTT GGGACTGGTT TAGGGCGGGG ACAAGACCAA GAACACAAGT 300

TTCCTTGTAC GGGAGAGAGG GAGGGGAGGA AATTGGAGAC CCCAGCACCC CCTTGCTCAC 360

TCTCTTGCTC ACAGTCCACG ATG 383